# EUROPEAN PATENT SPECIFICATION

(11) **EP 3 768 388 B1**
(45) Date of publication and mention of the grant of the patent: **17.04.2024**
(21) Application number: 18716528.7
(22) Date of filing: 21.03.2018
(51) Int. Cl.: A61Q 5/12, A61K 8/81, A61K 8/46

(54) **PROCESS FOR CONDITIONING OF KERATIN FIBERS**
VERFAHREN ZUR KONDITIONIERUNG VON KERATINFASERN
PROCÉDÉ DE CONDITIONNEMENT DES FIBRES KÉRATINIQUES

(43) Date of publication of application: 27.01.2021
(73) Proprietor: Kao Germany GmbH, 64297 Darmstadt (DE)
(72) Inventor: PICKER, Andreas, 64297 Darmstadt (DE); TIETJEN, Ilka, 64297 Darmstadt (DE); YOUNAS, Huma, 64297 Darmstadt (DE)
(74) Representative: Miller, Tobias
(86) International application number: PCT/EP2018/057209
(87) International publication number: WO 2019/179622

(56) References cited:
- WO-A1-2016/206738
- WO-A1-2016/206739
- WO-A1-2016/206740
- WO-A1-2017/198624

## Description

### Field of the invention

The present invention relates to the field of treating keratin fibers, preferably human keratin fibers, more preferably human hair, further more preferably to conditioning of aforementioned fibers. A method for treating keratin fibers, a kit-of-part, and a use is disclosed.

### Background of the invention

Since human beings started to think about beauty and their outward appearance, healthy, shiny, and brilliant hair was a sign of well-being, comfort, luxuriousness, and wealth. To achieve the above mentioned effects, people of former times used to treat their hair with acidic treatments such as acetic acid (vinegar). Nowadays, a huge variety of commercialized products are available under the categories conditioner, mask or treatment.

However, on the one hand most of the aforementioned products are based on conventional conditioning ingredients which rinse-off with the next hair wash with a conventional shampoo. Such conditioning treatments are disclosed in EP1479368 and conventional shampoos are found in Mintel # 4352781. On the other hand there is the consumers desire to apply conditioning products less frequently while maintaining a state of cosmetically healthy hair over an extended period of time. Especially consumers with damaged hair and environmentally-sensitive consumers wish to reduce their burden of applying a conditioning composition upon every shampooing of their hair.

### Summary of the invention

It has been unexpectedly found out by the inventors of the present invention that cationic polymers with a certain charge density remain longer and stronger deposited on the surface of the keratin fibers when the first contact with a cleansing composition upon the conditioning treatment comprises a limited amount of alkyl sulfate and alkyl ether sulfate surfactants, respectively. For the subsequent contacts with cleansing compositions this limitation of the first contact does not apply. Thus, the washfastness of the cationic polymers is increased and the healthy cosmetic properties of keratin fibers are maintained over several shampooing cycles. Such cosmetic properties are healthy feel, touch, suppleness, and non-charged state of the keratin fibers.

Therefore, the first object of the present invention is a method for treating keratin fibers, preferably human keratin fibers, more preferably human hair, as defined in claim 1.

### Detailed description of the invention

### Treatment process

Any of the compositions recited in the method claim 1 a) and/or b) and/or optionally c) may be left on the keratin fibers in a time range of 10 s to 600 s, preferably 30 s to 500 s, more preferably 60 s to 300 s. After this process time the rinsing-off steps are carried out as outlined above.

### Composition a)

The cationic polymers for the first non-cleansing composition a) have a charge density of at least 3 meq/g. The cationic charge density according to the present invention is calculated as the number of cationic charges per unit divided by molecular weight of the unit and multiplied by 1000 in order to express it as meq/g. It is to be noted that the reported charge density values in the following sections are to be understood as rounded values to one digit instead of exact numbers.

Suitable cationic polymers are:
(1) polymers known under the CTFA name Polyquaternium 16 and/or its salts which are marketed as Luviquat FC 550, and Luviquat HM 552 having a cationic charge density of 3.3 meq/g and 3.0 meq/g, respectively.
(2) polymers known under the CTFA name Polyquaternium 7 and/or its salts which is available with the trade name Merquat 550 having a charge density of 3.1 meq/g.
(3) polymers known under the CTFA name Polyquaternium 16 and/or its salts which are marketed as Luviquat Excellence having a cationic charge density of 6.1 meq/g.
(4) polymers known under the CTFA name Polymethacrylamidopropyltrimonium chloride or Polymethacrylamidopropyltrimonium chloride/acrylamide copolymer being sold under the tradename N-Durhance A-1000 and N-Durhance AA 2000, respectively, while having a cationic charge density of 4.8 meq/g.
(5) polymers sold under the CTFA name Polyquaternium 37 and/or its salts offered as Ultragel 300 having a charge density of 5.8 meq/g.

The preferred cationic polymer for the composition a) is Polyquaternium 37.

The concentration of cationic polymers with a charge density of at least 3 meq/g in the composition a) is in the range of 0.01 % to 10% by weight, preferably 0.05% to 8% by weight, more preferably in the range of 0.1 % to 6% by weight, further more preferably in the range of 0.2% to 2% by weight, calculated to the total of the composition a).

It is to be noted that for achieving the desired effect of the present invention, the presence of the aforementioned cationic polymers is not necessary in the compositions b) and optionally c). However, as it will be easily recognized by the skilled worker, the presence is not excluded in the compositions b) and c). Thus, the cationic polymers may be present in those compositions in a suitable quantity.

In one embodiment of the present invention the compositions b) and/or optionally c) are free of cationic polymers with a charge density of at least 3 meq/g.

The compositions a) and/or b) and/or c) preferably are aqueous compositions. The pH of the composition a) and/or b) and/or c) is/are in the range of 1 to 8, preferably in the range of 3 to 6.5, more preferably in the range of 4 to 6.

In an embodiment of the present invention the composition a) is non-aqueous. As solvent and/or dispersant for the cationic polymers a suitable organic solvent may be selected. Such solvents are, for example, primary alcohols such as ethanol, propanol, or isopropanol, and/or their mixtures, or mixtures of polyethylene glycols being liquid at room temperature and atmospheric conditions.

### Surfactants

The compositions a) and/or b) and/or c) comprise one or more surfactant(s), preferably selected from non-ionic, cationic, anionic and/or zwitterionic/amphoteric surfactants.

Suitable cationic surfactants are of quaternary ammonium structure according to the following general structure 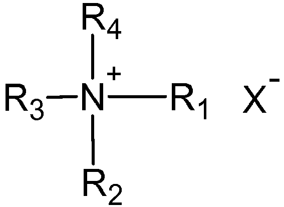
where R₁ is a saturated or unsaturated, branched or linear alkyl chain with C₈-C₂₂ or

   R₅ CO NH (CH₂)ₙ
where R₅ is saturated or unsaturated, branched or linear alkyl chain with C₇-C₂₁ atoms and n has typical value of 1-4 or

   R₆ CO O (CH₂)ₙ
where R₆ is saturated or unsaturated, branched or linear alkyl chain with C₇-C₂₁ atoms and n has typical value of 1-4, and
R₂ is unsaturated or saturated, branched or linear alkyl chain with C₁-C₂₂ atoms or

   R₅ CO NH (CH₂)ₙ

   or

   R₆ CO O (CH₂)ₙ
where R₅, R₆ and n are same as above.

R₃ and R₄ have an alkyl chain with C₁ to C₄, and X⁻ is typically chloride, bromide, or methosulfate.

Typical examples of those ingredients are cetyl trimethyl ammonium chloride, stearyl trimonium chloride, dipalmitoyl dimonium chloride, distearyl dimethyl ammonium chloride, stearamidopropyl trimethyl ammonium chloride, dioleoylethyl dimethyl ammonium methosulfate, dioleoylethyl hydroxyethylmonium methosulfate, behenyl trimethyl ammonium chloride, and/or their mixtures.

Suitable cationizable surfactants are surfactants which carry one or more chemical group(s) that is/are at least at some point non-ionic at pH above 7, but which is/are at least partially positively charged at a pH under 7. Such groups are, for example, primary, secondary, and tertiary amino groups. It is well known to the skilled reader that the aforementioned groups are becoming ammonium groups at a pH below 7.

Suitable cationizable surfactants are, for example, according to the following general structure 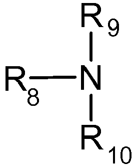 where R₈ is a saturated or unsaturated, straight or branched alkyl chain, optionally modified with ethoxylate and/or propoxylate groups, with C₁₂ to C₂₂, R₉ is selected from H or straight or branched alkyl with C₁ to C₄, and R₁₀ is selected from H or straight or branched alkyl with C₁ to C₄.

Suitable compounds according to this structure are, for example, dodecylamine, tridecylamine, tetradecylamine, pentadecylamine, stearylamine, octylamine, oleylamine, behenylamine, stearyl methylamine, stearyl dimethylamine, octyl methylamine, octyl dimethylamine, behenyl methylamine, behenyl dimethylamine, stearyl ethylamine, stearyl ethylamine, octyl ethylamine, octyl ethylamine, behenyl ethylamine, behenyl ethylamine, stearyl propylamine, stearyl dipropylamine, octyl propylamine, octyl dipropylamine, behenyl propylamine, behenyl dipropylamine and/or their salts and/or their mixtures. The aforementioned compounds may be modified with ethoxylate and/or propoxylate groups.

Further suitable cationizable surfactants are known as alkyl amido alkyl amine surfactants and/or their salt(s) and are according to the following general structure 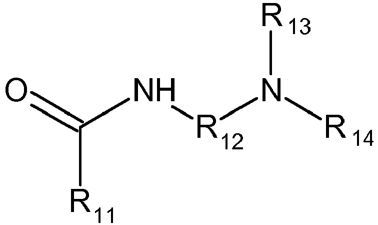 where R₁₁ is a saturated or unsaturated, straight or branched alkyl chain with C₁₁ to C₂₁, R₁₂ is a straight or branched alkyl chain with C₁ to Ce, R₁₃ and R₁₄ may be the same of different selected from H and straight or branched alkyl chain with C₁ to C₄.

Suitable compounds according to this definition are, for example, cocamidopropyl dimethylamine, stearamidopropyl dimethylamine, behenamidopropyl dimethylamine, and/or their salt(s).

The preferred cationic surfactant(s) is/are selected from quaternary ammonium surfactants and/or alkylamidoalkyl amine surfactants.

Suitable amphoteric/zwitterionic surfactants may be selected from compounds according to the general structure(s) 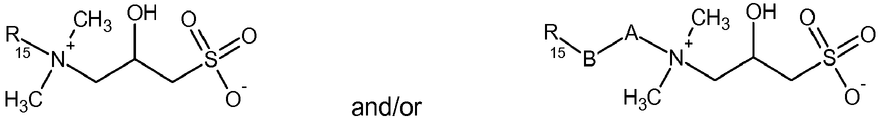 wherein R₁₅ is a straight or branched, saturated or unsaturated, substituted or unsubstituted alkyl chain with a carbon number of C₁₀ to C₂₂, preferably R₁₅ is a straight alkyl chain with a carbon number of C₁₀ to C₁₆, A is a straight alkyl chain with a carbon number of C₁ to C₆ or a branched alkyl chain with a carbon number of C₃ to C₆, preferably A is a linear alkyl chain with a carbon number of C₃, and B is an amide or an ester group.

Suitable compounds are known as hydroxysultaine surfactants, such as cocoamidopropyl hydroxysultaine, laurylamidopropyl hydroxysultaine, erucamidopropyl hydroxysultaine, lauryl hydroxysultaine, and cocoyl hydroxysultaine, and/or their salt(s).

Further suitable amphoteric/zwitterionic surfactants are of betaine type. Suitable compounds may be selected from alkyl betaines and/or alkylamido betaines. A preferred compound selected from alkyl betaines is lauryl betaine. A preferred compound selected from alkylamido betaines is cocamidopropyl betaine. The disclosure also relates to the salts of the compounds.

The preferred amphoteric/zwitterionic surfactant(s) is/are selected from alkylamido betaines and/or alkylamidoalkyl betaine surfactants.

Suitable nonionic surfactants are alkyl polyglycosides according to the general structure:

R₂₃O(R₂₄O)tZₓ

Wherein Z denotes a carbohydrate with C₅ to C₆, R₂₃ is an alkyl group with C₈ to C₁₈, R₂₄ is methyl, ethyl or propyl, t ranges from 0 to 10, and x ranges from 1 to 5. Suitable compounds according to this structure are C₉-C₁₁ alkylpolyglycoside, the structures disclosed in EP-A 70 074, and JP 2015-123019A.

The preferred compounds according to the structure of above are decyl glucoside, lauryl glucoside, and coco glucoside, and the most preferred one is decyl glucoside.

Further suitable examples for non-ionic surfactants are N-alkylpolyhydroxyalkylamide type surfactants according to the following general formula: 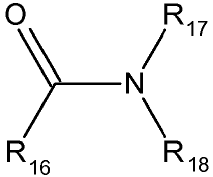 wherein R₁₆ is a linear or branched, saturated or unsaturated alkyl chain with C₁₁ to C₂₁, R₁₇ is linear or branched alkyl, or linear or branched hydroxyalkyl with C₁ to C₄, and R₁₈ is a linear or branched polyhydroxyalkyl chain with C₃ to C₁₂ and 3 to 10 hydroxyl groups.

Such compounds are disclosed in cosmetic compositions in WO96/27366 and their synthesis is disclosed in US1985424, US2016962, US2703798, and WO92/06984.

The preferred N-alkylpolyhydroxyalkylamide type surfactants have the following structure: 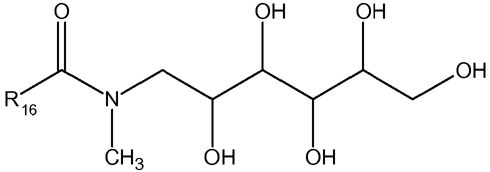 where R₁₆ has the same denotation as above for the general structure of N-alkylpolyhydroxyalkylamide type surfactants. The preferred surfactants as displayed above are known as N-methyl-N-acylglucamides.

The most preferred N-alkylpolyhydroxyalkylamide type surfactants are selected from lauroyl/myristoyl methyl glucamide and coco methyl glucamide.

Further suitable examples for non-ionic surfactants are fatty alcohol ethoxylates of the following general structure

R₂₅ (OCH₂CH₂)ₙ₄ OH

wherein R₂₅ is straight or branched, saturated or unsaturated alkyl chain which may be synthetic or natural with a C chain length in the range of 8 to 40, preferably 9 to 30 and more preferably 9 to 24 and n4 is a number in the range of 5 to 40, preferably 9 to 30.

Non-limiting suitable examples of the fatty alcohol ethoxylates are C9-11 Pareth-6, C9-11 Pareth-8, C9-15 Pareth-8, C11-13 Pareth-9, C11-13 Pareth-10, C11-15 Pareth-5, C11-15 Pareth-7, C11-15 Pareth-9, C11-15 Pareth-12, C11-15 Pareth-15, C11-15 Pareth-20, C11-15 Pareth-30, C11-15 Pareth-40, C11-21 Pareth-10, C12-13 Pareth-5, C12-13 Pareth-6, C12-13 Pareth-7, C12-13 Pareth-9, C12-13 Pareth-10, C12-13 Pareth-15, C12-13 Pareth-23, C12-14 Pareth-5, C12-14 Pareth-7, C12-14 Pareth-9, C12-14 Pareth-11, C12-14 Pareth-12, C12-15 Pareth-5, C12-15 Pareth-7, C12-15 Pareth-9, C12-15 Pareth-10, C12-15 Pareth-11, C12-15 Pareth-12, C12-16 Pareth-5, C12-16 Pareth-7, C12-16 Pareth-9, C13-15 Pareth-21, C14-15 Pareth-7, C14-15 Pareth-8, C14-15 Pareth-11, C14-15 Pareth-12, C14-15 Pareth-13, C20-22 Pareth-30, C20-40 Pareth-10, C20-40 Pareth-24, C20-40 Pareth-40, C20-40 Pareth-95, C22-24 Pareth-33, Beheneth-5, Beheneth-10, Beheneth-15, Beheneth-20, Beheneth-25, Beheneth-30, Ceteareth-5, Ceteareth-6, Ceteareth-7, Ceteareth-10, Ceteareth-11, Ceteareth-12, Ceteareth-15, Ceteareth-20, Ceteareth-25, Ceteareth-30, Ceteareth-35, Ceteareth-40, Laureth-5, Laureth-10, Laureth-15, Laureth-20, Laureth-25, Laureth-30, Laureth-40, Myreth-5, Myreth-10, Ceteth-5, Ceteth-10, Ceteth-15, Ceteth-20, Ceteth-25, Ceteth-30, Ceteth-40, Oleth-5, Oleth-10, Oleth-15, Oleth-20, Oleth-25, Oleth-30, Oleth-40, Steareth-5, Steareth-10, Steareth-15, Steareth-20, Steareth-25, Steareth-30, Steareth-35, and Steareth-40. They may also be comprised in the compositions as a mixture of more than one surfactant.

Further suitable nonionic surfactants are polypropylene glycol ethers of fatty alcohol according to general structure

R₂₅ (OCH₂-CH₂-CH₂)ₙ₅ OH

wherein R₂₅ is straight or branched, saturated or unsaturated fatty alcohol which may be synthetic or natural with a C chain length in the range of 8 to 40, preferably 9 to 30 and more preferably 9 to 24 and n5 is a number in the range of 1 to 40, preferably 3 to 30.

Suitable non-limiting examples are PPG-3 Caprylyl ether, PPG-5 Caprylyl ether, PPG-10 Caprylyl ether, PPG-10 Cetyl ether, PPG-20 Cetyl ether, PPG-28 Cetyl ether, PPG-30 Cetyl ether, PPG-7 Lauryl ether, PPG-10 Lauryl ether, PPG-10 Oleyl ether, PPG-20 Oleyl ether, PPG-23 Oleyl ether, PPG-30 Oleyl ether, PPG-11 Stearyl ether and PPG-15 Stearyl ether.

Further suitable nonionic surfactants are polyethylene glycol fatty acid esters of the following general structure

R₂₆ C(O) (OCH₂CH₂)ₙ₆ OH

wherein R₂₆ is straight or branched, saturated or unsaturated alkyl group which may be synthetic or natural with a C chain length in the range of 7 to 39, preferably 9 to 29 and more preferably 9 to 23 and n6 is a number in the range of 5 to 40, preferably 9 to 30.

Suitable non-limiting examples are PEG-8 Behenate, PEG-8 Caprate, PEG-8 Caprylate, PEG-5 Cocoate, PEG-8 Cocoate, PEG-9 Cocoate, PEG-10 Cocoate, PEG-15 Cocoate, PEG-6 Isopalmitate, PEG-6 Isostearate, PEG-8 Isostearate,
PEG-9 Isostearate, PEG-10 Isostearate, PEG-12 Isostearate, PEG-20 Isostearate, PEG-30 Isostearate, PEG-40 Isostearate, PEG-6 Laurate, PEG-8 Laurate, PEG-9 Laurate, PEG-10 Laurate, PEG-12 Laurate, PEG-14 Laurate, PEG-20 Laurate, PEG-30 Laurate, PEG-8 Myristate, PEG-20 Myristate, PEG-5 Oleate, PEG-6 Oleate, PEG-7 Oleate, PEG-8 Oleate, PEG-9 Oleate, PEG-10 Oleate, PEG-11 Oleate, PEG-12 Oleate, PEG-15 Oleate, PEG-20 Oleate, PEG-30 Oleate, PEG-32 Oleate, PEG-6 Palmitate, PEG-18 Palmitate, PEG-20 Palmitate, PEG-5 Stearate, PEG-6 Stearate, PEG-7 Stearate, PEG-8 Stearate, PEG-9 Stearate, PEG-10 Stearate, PEG-12 Stearate, PEG-14 Stearate, PEG-15 Stearate, PEG-20 Stearate, PEG-25 Stearate, PEG-30 Stearate, PEG-35 Stearate and PEG-40 Stearate.

Further suitable nonionic surfactants are polypropylene glycol fatty acid esters of the following general structure

R₂₇ C(O) (OCH₂-CH₂-CH₂)ₙ₈ OH

wherein R₂₇ is straight or branched, saturated or unsaturated alkyl group which may be synthetic or natural with a C chain length in the range of 7 to 39, preferably 9 to 29 and more preferably 9 to 23 and n8 is a number in the range of 1 to 40, preferably 9 to 30.

Suitable non-limiting examples are PPG-15 Isostearate, PPG-9 Laurate, PPG-26 Oleate and PPG-36 Oleate.

Further suitable nonionic surfactants are polyethylene glycol and polypropylene glycol ether of fatty alcohols of the following general structure

R₂₈ (OCH₂-CH₂-CH₂)ₙ₉ (OCH₂CH₂)ₙ₁₀ OH

wherein R₂₈ is straight or branched, saturated or unsaturated alkyl group which may be synthetic or natural with a C chain length in the range of 7 to 39, preferably 9 to 29 and more preferably 9 to 23 and n9 and n10 may be the same or different and are a number in the range of 1 to 40.

Further suitable nonionic surfactants are ethoxylated vegetable oils. Well-known and commonly used examples are ethoxylated castor oil such as PEG-40 hydrogenated castor oil or and PEG-60 hydrogenated castor oil.

The preferred non-ionic surfactant(s) are selected from alkyl polyglycoside(s), ethoxylated and/or propoxylated fatty alcohols, ethoxylated and/or propoxylated vegetable oils, preferably they are selected from C₈-C₂₂ alkyl polyglycoside(s), more preferably they are selected from decyl glucoside, lauryl glucoside, and coco glucoside, and further more preferably it is coco glucoside.

The total concentration of surfactants of the compositions b) and/or c) is in the range of 5% to 50% by weight, preferably 8% to 35% by weight, more preferably in the range of 12% to 25% by weight, further more preferably in the range of 14% to 20% by weight, calculated to the total of each compositions b) and c). As the composition a) is a non-cleansing composition, but might require some surfactants, the total concentration of surfactants in the composition a) is below 3% by weight, preferably below 2% by weight, calculated to the total of the composition a).

In one embodiment of the present invention the composition c) comprises alkyl sulfate and/or alkyl ether sulfate surfactants at a concentration of more than 50% by weight, preferably more than 90% by weight, calculated to the total surfactant concentration of the composition c).

It is to be noted that the skilled person can combine the surfactants from the classes of above to create suitable surfactant mixtures.

### Suitable cosmetic forms

The compositions a) and/or b) and/or c) may be in any suitable cosmetic form such as solution, suspension, thickened gel, or emulsion.

For preparation of a thickened gel or emulsion, the compositions a) and/or b) and/or c) may comprise a polymeric thickening polymer, preferably an anionic and/or non-ionic thickening polymer, more preferably a carbohydrate-based non-ionic thickening polymer.

In a preferred embodiment of the present invention, the composition comprises one or more thickening polymers selected from anionic, nonionic, cationic and amphoteric polymers, preferably selected from polymers resulting in a solution and/or dispersion with a viscosity of at least 500 mPa•s measured at a polymer concentration of 1% by weight in water and at 20°C with a Brookfield viscometer and spindle 4, such as at 10 rpm for 1 min.

Suitable polymers are cellulose polymers, alginates, polysaccharides and acrylic acid polymers, preferably methyl cellulose, ethyl cellulose, hydroxyethylcellulose, methylhydroxyethylcellulose, methylhydroxypropylcellulose, carboxymethyl cellulose, alginic acids, sodium alginates, ammonium alginates, calcium alginates, gum arabic, guar gum or xanthan gum, dehydroxanthan gum or acrylic acid polymers known with the CTFA adopted name Carbomer and its derivatives.

The preferred polymers are hydroxyethylcellulose, xanthan gum, and polymeric anionic thickeners, namely Carbomer and its derivatives.

Of particular advantageous use are thickeners which are commonly known as associative thickeners. Preferred are copolymers and/or crosspolymers which comprise an acrylate and/or methacrylate monomer unit and at least one more hydrophobic unit such as alkyl chains. Examples are acrylates/c10-30 alkyl acrylate crosspolymer, acrylates/steareth-20 methacrylate copolymer, acrylates/stearyl acrylate/dimethicone methacrylate copolymer, acrylates/beheneth-25 methacrylate copolymer, acrylates/lauryl acrylate/stearyl acrylate/ ethylamine oxide methacrylate copolymer.

The compositions a) and/or b) and/or c) preferably comprise thickening agents at a total concentration in the range of 0.1% to 5%, preferably, 0.2% to 3%, more preferably 0.25% to 2.5% and most preferably 0.3% to 2% by weight calculated to the total of each composition.

The compositions a) and/or b) and/or c) may have a viscosity in the range of 1 mPas to 50,000 mPas, preferably in the range of 100 mPas to 25,000 mPas, more prefably in the range of 1,000 to 15,000 mPas, measured with a Brookfield viscosimeter using spindle #4 at 20°C under atmospheric conditions.

### Hydrophobic compounds

The compositions a) and/or b) and/or c) comprise one or more hydrophobic compound(s), preferably selected from fatty alcohols, fatty acid ester, silicone oils, aminated silicones, and mineral oil.

Suitable fatty alcohols having a linear or branched, saturated or unsaturated carbon chain length of C₁₂-C₂₂ according to the present invention are, for example, lauryl alcohol, tridecyl alcohol, myristyl alcohol, pentadecyl alcohol, cetyl alcohol, palmitoleyl alcohol, heptadecyl alcohol, stearyl alcohol, oleyl alcohol, nonadecyl alcohol, arachidyl alcohol, behenyl alcohol, and/or their mixtures, in particular cetearyl alcohol.

Fatty alcohols within the meaning of the present invention are not considered as surfactants.

Suitable fatty acids are saturated or unsaturated fatty acids with or without a hydroxyl group. Suitable are myristoleic acid, palmitoleic acid, oleic acidlinoleic acid, arachidonic acid, and/or their mixtures.

Suitable compounds according to the general structure from above:
are isopropyl palmitate, isopropyl myristate, octyl palmitate, isocetyl palmitate, octyl stearate, oleyl oleate, ethylhexyl hydroxystearate, myristyl myristate, behenyl behenate, and/or their mixtures.

Suitable vegetable oils are jojoba oil, avocado oil, sunflower seed oil, walnut oil, peanut oil, olive oil, rapeseed oil, cottonseed oil, palm oil, sesame oil, soybean oil, coconut oil, safflower oil, almond oil, macadamia nut oil, grapefruit seed oil, lemon kernel oil, orange kernel oil, apricot kernel oil, castor oil, argan oil, tamanu oil, and/or their mixtures.

Suitable petrolatum-based products are liquid paraffins, especially paraffinum perliquidum and paraffinum subliquidum, and mineral oil, in particular white mineral oil, and/or their mixtures.

Suitable silicones are dimethylpolysiloxanes, and modified silicones (for example, amino-modified silicones, fluorine-modified silicones, alcohol-modified silicones, polyether-modified silicones, epoxy-modified silicones, or alkyl-modified silicones), but dimethylpolysiloxane, polyether-modified silicones and amino-modified silicones are preferred. Amino-modified silicones are commonly known under their CTFA name amodimethicone.

The dimethylpolysiloxane may be any cyclic or non-cyclic dimethylsiloxane polymer, and examples thereof include SH200 series, BY22-019, BY22-020, BY11-026, B22-029, BY22-034, BY22-050A, BY22-055, BY22-060, BY22-083, FZ-4188 (all by Dow Corning Toray Co., Ltd.), KF-9008, KM-900 series, MK-15H, and MK-88 (all by Shin-Etsu Chemical Co., Ltd.).

The polyether-modified silicone may be any silicone having a polyoxyalkylene group, and the group constituting the polyoxyalkylene group may be an oxyethylene group or an oxypropylene group. More specific examples include KF-6015, KF-945A, KF-6005, KF-6009, KF-6013, KF-6019, KF-6029, KF-6017, KF-6043, KF-353A, KF-354A, KF-355A (all by Shin-Etsu Chemical Co., Ltd.), FZ-2404, SS-2805, FZ-2411, FZ-2412, SH3771M, SH3772M, SH3773M, SH3775M, SH3749, SS-280X series, BY22-008 M, BY11-030, and BY25-337 (all by Dow Corning Toray Co., Ltd.).

Specific examples of suitable commercially available amodimethicone oils such as SF8452C, SS-3551 (all by Dow Corning Toray Co., Ltd.), KF-8004, KF-867S, and KF-8015 (all by Shin-Etsu Chemical Co. , Ltd.), and amodimethicone emulsions such as SM8704C, SM8904, BY22-079, FZ-4671, and FZ-4672 (all by Dow corning Toray Co., Ltd.).

Further suitable hydrophobic compounds are linear and/or cyclic non-aminated silicones and/or non-aminated siliconols.

Suitable non-aminated silicones and/or non-aminated siliconols are dimethicone, dimethiconol, polydimethylsiloxane (DC fluid ranges from Dow Corning), arylated silicones such as phenyl methicone, phenyl trimethicone, diphenyl dimethicone, diphenylsiloxy phenyl trimethicone, tetramethyl tetraphenyl trisiloxane, triphenyl trimethicone, and trimethyl pentaphenyl trisiloxane, aqueous emulsions of divinyldimethicone/dimethicone copolymer, preferably with a viscosity of higher than 1 × 10⁸ mm²/s, more preferably higher than 1.1 × 10⁸ mm²/s measured at 0.01 Hz and at 20°C with a Brookfield viscometer and an appropriate spindle.

The composition may further comprise aminosilicone(s), preferably selected from a compound according to the general structure

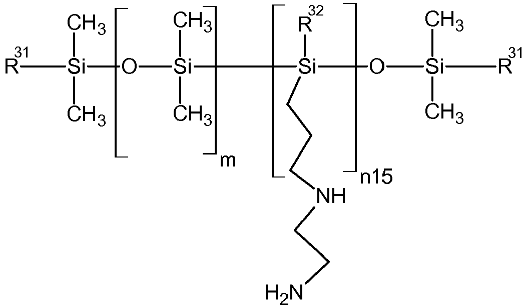

Wherein R³¹ is selected from OH, OCH₃, and/or O-Si-(CH₃)₃, R³² is selected from CH₃, OCH₃, O-(Si-(CH₃)₂)x-R³³, and/or O-Si-(CH₃)₃, with the provision that if R³¹ or R³² are selected from O-Si-(CH₃)₃, then all other R³² or R³³ are selected from O-Si-(CH₃)₃ and/or OCH₃, and x is an integer from 1 to 200. m and n15 are integer numbers independently of each other and in the range of 1 to 200. Special reference is made to the aminosilicones sold by Wacker Corporation under the trade name Belsil ADM 6102E and Belsil ADM 8020VP, the ones sold by by Shin-Etsu Corp. under the trade name X-52-2265, and the ones sold by Dow Corning Corp. under the trade name DC 969.

The compositions a) and/or b) and/or c) may comprise hydrophobic compounds at a total concentration in the range of 0.01% to 10% by weight, preferably 0.05% to 8% by weight, more preferably 0.1 to 6% by weight, calculated to the total of each composition.

In case that cationic silicone compounds are added to the compositions, the concentration of cationic silicones is in the range of 0.01% to 1% by weight, preferably 0.1% to 0.5% by weight, calculated to the total of each composition.

The compositions a) and/or b) and/or c) may comprise cationic polymers with a charge density of less than 3 meq/g.

Typical examples of those are Polyquaternium 1, Polyquaternium 2, Polyquaternium 4, Polyquaternium 5, Polyquaternium 8, Polyquaternium 9, Polyquaternium 10, Polyquaternium 11, Polyquaternium 12, Polyquaternium 13, Polyquaternium 14, Polyquaternium 15, Polyquaternium 17, Polyquaternium 18, Polyquaternium 19, Polyquaternium 20, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium 39, Polyquaternium 42, Polyquaternium 43, Polyquaternium 44, Polyquaternium 45, Polyquaternium 46, Polyquaternium 47, Polyquaternium 48, Polyquaternium 49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86, and Polyquaternium 87.

It has further been found out that especially those of cationic cellulose type polymers known as Polymer JR type from Amerchol such as Polyquaternium 10 or cationic galactomannans such as cationic guar gum known with trade name Jaguar from Rhone-Poulenc which are chemically for example guar hydroxypropyl trimonium chloride and cationic tara gum and its derivatives known with INCI name Caesalpinia spinosa hydroxypropyltrimonium chloride, are preferred ones. Furthermore, chitosan and chitin can also be included in the compositions as cationic natural polymers. In this context reference is also made to the cationic polymers disclosed in DE 25 21 960, DE 28 11 010, 30 44 738 and DE 32 17 059, as well as to the products described in EP-A 337 354 on pages 3 to 7. It is also possible to use mixtures of various cationic polymers.

The most preferred cationic polymers with a charge density of less than 3 meq/g are those of cationic cellulose derivatives, cationic guar gum derivatives, cationic Caesalpinia spinosa gum derivatives. The cationic polymers also include the quaternized products of graft polymers from organopolysiloxanes and polyethyl oxazolines described in EP-A 524 612 and EP-A 640 643.

Compositions may comprise cationic polymers with a charge density of less than 3 meq/g at a concentration of 0.01 to 5%, preferably 0.02 to 4%, more preferably 0.05 to 3% and most preferably 0.1 to 2.5% by weight, calculated to the total of the composition.

In cases where a cationic silicone compound is added to the composition, the narrower concentration limitation for silicone compounds applies to the cationic silicone compounds as well.

The compositions a) and/or b) and/or c) may further comprise one or more UV filters which may be selected from water soluble ones as well as oils soluble ones. The oil soluble UV filter are more preferred ones as they show no interaction with the cationic quaternary ammonium polymers. Non-limiting examples are 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher, and/or polysilicone-15.

The total UV filter concentration may be in the range of 0.01% to 1 % by weight, calculated to the total of the composition.

The compositions a) and/or b) and/or c) may comprise organic solvents with a concentration of up to 10% by weight, calculated to the total of the composition. The organic solvents are selected from C1 to C4 linear or C3 to C4 branched alcohols or aromatic alcohols. Suitable C1 to C4 linear alcohols are ethanol, n-propanol, and n-butanol, glycerol, propylene glycol, butylene glycol and suitable C3 to C4 branched alcohols are iso-propanol, tert-butanol, iso-butanol. Aromatic alcohols are for example phenol, phenoxyethanol, benzyl alcohol, 2-phenylethanol, 2-benzoyloxyethanol. The skilled I n the art will recognize that some of the aforementioned organic solvents can act as preservatives.

The following examples are to illustrate the invention, but not to limit it.

### EXAMPLES

### Example 1

The following compositions were prepared by conventional mixing and dissolution techniques:

### First non-cleansing composition a)

| | **% by weight** |
|---|---|
| Polyquaternium-37 | 0.5% |
| Phosphoric acid | ad pH 5.5 |
| Water | ad 100 |

### Third cleansing composition c)

| | **% by weight** |
|---|---|
| Sodium laureth sulfate | 10.0 |
| Phosphoric acid | ad pH 5.5 |
| Water | ad 100 |

### Second cleansing compositions b)

| **Ingredients** | **Inventive composition A** | **Inventive composition B** | **Inventive composition C** | **Comparative composition A** |
|---|---|---|---|---|
| | % by weight | | | |
| **Sodium laureth sulfate** | - | 2.5 | 5.0 | 10.0 |
| **Cocamidopropyl Betaine** | 11.7 | 10.0 | 8.3 | 4.8 |
| **Sodium Lauroyl Sarcosinate** | 1.8 | 1.5 | 1.2 | 0.7 |
| **Coco-Glucoside** | 3.5 | 3.0 | 2.5 | 1.5 |
| **Phosphoric acid** | Ad pH 5.5 | | | |
| **Water** | Ad 100 | | | |
| | | | | |
| **Total surfactant concentration** | 17.0 | 17.0 | 17.0 | 17.0 |
| **% by weight of sodium laureth sulfate to total surfactant concentration** | 0 | 14.7 | 29.4 | 58.8 |

Human hair streaks (Caucasian. 21 cm long) were purchased from Fischbach + Miller Haar, Laupheim, Germany. The hair streaks were bleached twice with a commercial bleach available under the brand name Goldwell SilkLift Control. The hair streaks were then oxidatively dyed with hair color available under the brand name Goldwell Topchic 7RR. The streaks were thoroughly washed and blow-dried.

At first, 1 g of the first non-cleansing composition a) from above was applied onto each hair streak and left for 5 min. The hair streaks were then rinsed-off with warm water for 60 s, then the second cleansing compositions b) from above were applied to hair streaks and massaged for 60 s. The hair streaks were then thoroughly rinsed off and blow-dried. Suppleness of hair was determined at this time point and is defined as 0 washes. As a last step each hair streak was washed with the second cleansing composition c) for 3, 5, and 10 times and hair suppleness was determined after these time points.

The suppleness of the hair was measured with a zwick machine and expressed as the force (in mN) to pull a streak through the rods of the machine. The machine is commercially available from Zwick/Roell Company and includes a manual. It was used according to its manual. Briefly, the streaks were placed between the metal rods offset in a given distance horizontally (2 cm) and vertically (8 cm) and the force needed to pull the streaks through the metal rods at a given rate (5 cm/min) was measured. All values reported in the table below are averages of at least 6 readings.

**The following results were obtained:**

| **Time points** | **Inventive comp A [mN]** | **Inventive comp B [mN]** | **Inventive comp C [mN]** | **Comparative comp A [mN]** |
|---|---|---|---|---|
| **0 washes** | 739 | 631 | 608 | 1013 |
| **3 washes** | 512 | 569 | 586 | 1238 |
| **5 washes** | 657 | 866 | 683 | 1700 |
| **10 washes** | 495 | 633 | 576 | 1665 |

As was revealed by the suppleness measurements, the force values for hair streaks treated with the inventive compositions A-C scattered in the range of 608 mN to 739 mN prior to washing cycles , whereas the hair streak treated with the comparative composition had force values above 1,000 mN. This difference between inventive and comparative composition prevailed over the washing cycles and the higher suppleness for the inventive composition lasted over 10 washes. Thus, the presence of more than 50% by weight of sodium laureth sulfate, calculated to the total of the surfactant concentration, as being the first contact with a cleansing composition, immediately diminished the conditioning effect.

### Example 2

The same compositions of example 1 were employed and the hair streaks were pretreated in the same manner.

However, in contrast to example 1 the composition a) was not rinsed off and the cleansing compositions b) were applied to the hair streaks.

Suppleness measurements revealed the same data trend as presented in example 1.

Thus, the obtained effect is independent of a rinsing step between the application of compositions a) and b).

### Example 3

The same compositions of example 1 were employed and the hair streaks were pretreated in the same manner.

However, in contrast to examples 1 and 2 the compositions a) and the cleansing compositions b) were mixed prior to the application onto hair in a weight ratio of 1:1. This mixture was then applied onto hair, allowed to process for 5 min, and then rinsed off. The composition c) was applied as laid out in example 1.

Suppleness measurements revealed the same data trend as presented in examples 1 and 2.

Thus, the obtained effect is independent of a 2-step application of the compositions a) and b).

The following examples are within the scope of the present invention.

### Example 4

### First non-cleansing composition a)

| | % by weight |
|---|---|
| Behenamidopropyl dimethylamine | 0.50 |
| Polyquaternium 16 | 0.75 |
| Polyquaternium 37 | 0.25 |
| Cetearyl alcohol | 1.5 |
| Lactic acid | ad pH 6.0 |
| Water | ad 100.0 |

The composition from above may also comprise Polyquaternium 6, Polyquatenrium 7, Polyquaternium 16, or Polymethacrylamidopropyltrimonium chloride as the only cationic polymer in a concentration range of 0.1% by weight to 10% by weight.

### Second cleansing composition b)

| | % by weight |
|---|---|
| Cocoamidopropyl betaine | 5.0 |
| Sodium lauroyl glutamate | 2.5 |
| Alkylpolyglycosides | 3.0 |
| Ceteareth-10 | 1.0 |
| Sodium laureth sulfate | 1.5 |
| Hydroxyethylcellulose | 0.75 |
| Lactic acid | ad pH 5.0 |
| Water | ad 100.0 |

### Third cleansing composition c)

| | % by weight |
|---|---|
| Sodium lauryl sulfate | 15.0 |
| Alkylpolyglycosides | 3.0 |
| Cocoyl betaine | 1.0 |
| Malic acid | ad pH 5.5 |
| Water | ad 100.0 |

## Claims

1. A method for treating keratin fibers, preferably human keratin fibers, more preferably human hair, **characterized in that** it comprises the following compositions:
a) a first non-cleansing composition comprising one or more cationic polymer(s) and/or their salt(s) with a charge density of at least 3 meq/g, having a total concentration of surfactants below 3% by weight, preferably below 2% by weight, calculated to the total weight of composition a),
b) a second cleansing composition comprising cleansing surfactants at a total concentration in the range of 5% to 50% by weight, calculated to the total of the cleansing composition, and comprising less than 50% by weight, preferably less than 30% by weight, more preferably less than 15% by weight of alkyl sulfate and alkyl ether sulfate surfactants, calculated to the total surfactant amount of the cleansing composition,
c) optionally a third cleansing composition comprising cleansing surfactants at a total concentration in the range of 5% to 50% by weight, calculated to the total of the composition,
wherein the compositions are applied in the following order:
i) at first applying to keratin fibers the composition of a), rinsing it off, at second applying composition of b) with a time delay of maximum 12 h upon application of a), rinsing it off, and optionally at third then applying the composition of c) followed by rinsing it off,
ii) at first applying to keratin fibers the composition of a), at second applying the composition of b), rinsing-off the compositions a) and b), and optionally at third applying the composition of c) followed by rinsing it off,
iii) at first mixing the compositions a) and b) directly prior to application onto keratin fibers, applying the mixture to keratin fibers and rinsing-off the mixture, and optionally at second applying the composition of c) followed by rinsing it off.

2. The method according to claim 1 **characterized in that** one or more cationic polymer(s) of composition a) is/are selected from Polyquaternium 6, Polyquaternium 7, Polyquaternium 16, Polyquaternium 37, and/or their salt(s), Polymethacrylamidopropyltrimonium chloride, and Polymethacrylamidopropyltrimonium chloride/acrylamide copolymer, preferably it is Polyquaternium 37.

3. The method according to the claims 1 and/or 2 **characterized in that** the concentration of cationic polymers with a charge density of at least 3 meq/g in the composition a) is in the range of 0.01% to 10% by weight, preferably 0.05% to 8% by weight, more preferably in the range of 0.1% to 6% by weight, further more preferably in the range of 0.2% to 2% by weight, calculated to the total of the composition a).

4. The method according to any of the preceding claims **characterized in that** the pH of the composition a) and/or b) and/or c) is/are in the range of 1 to 8, preferably in the range of 3 to 6.5, more preferably in the range of 4 to 6.

5. The method according to any of the preceding claims **characterized in that** the compositions a) and/or b) and/or c) comprise one or more surfactant(s), preferably selected from non-ionic, cationic, anionic and/or zwitterionic/amphoteric surfactants.

6. The method according to any of the preceding claims **characterized in that** the amphoteric/zwitterionic surfactants are selected from alkylamido betaines and/or alkylamidoalkyl betaine and/or sultaine surfactants.

7. The method according to any of the preceding claims **characterized in that** the cationic surfactant is selected from quaternary ammonium surfactants and/or alkylamidoalkyl amine surfactants.

8. The method according to any of the preceding claims **characterized in that** the total concentration of surfactants of the compositions b) and/or c) is in the range of 8% to 35% by weight, preferably in the range of 12% to 25% by weight, more preferably in the range of 14% to 20% by weight, calculated to the total of each compositions b) and c), and the total concentration of surfactants in the composition a) is below 3% by weight, preferably below 2% by weight, calculated to the total of the composition a).

9. The method according to any of the preceding claims **characterized in that** cationic charge density of the cationic polymer of composition a) is at least 4.5 meq/g, preferably at least 5.5 meq/g.

10. The method according to any of the preceding claims **characterized in that** the compositions b) and/or optionally c) are free of cationic polymers with a charge density of at least 3 meq/g.

11. The method according to any of the preceding claims **characterized in that** the compositions a) and/or b) and/or optionally c) comprise one or more hydrophobic compound(s), preferably selected from fatty alcohols, fatty acid ester, silicone oils, aminated silicones, and mineral oil.

12. The method according to any of the preceding claims **characterized in that** the composition c) comprises alkyl sulfate and/or alkyl ether sulfate surfactants at a concentration of more than 50% by weight, preferably more than 90% by weight, calculated to the total surfactant concentration of the composition c).

## Patentansprüche

1. Verfahren zur Behandlung von Keratinfasern, vorzugsweise menschlichen Keratinfasern, besonders bevorzugt menschlichen Haaren, **dadurch gekennzeichnet, dass** es die folgenden Zusammensetzungen umfasst:
a) eine erste nicht reinigende Zusammensetzung, die ein oder mehrere kationische(s) Polymer(e) und/oder ihr(e) Salz(e) mit einer Ladungsdichte von mindestens 3 meq/g enthält, mit einer Gesamtkonzentration an Tensiden unter 3 Gew.-%, vorzugsweise unter 2 Gew.-%, berechnet auf das Gesamtgewicht der Zusammensetzung a),
b) eine zweite Reinigungszusammensetzung, umfassend Reinigungstenside in einer Gesamtkonzentration im Bereich von 5 Gew.-% bis 50 Gew.-%, berechnet auf die Gesamtmenge der Reinigungszusammensetzung, und umfassend weniger als 50 Gew.-%, vorzugsweise weniger als 30 Gew.-%, noch bevorzugter weniger als 15 Gew.-% an Alkylsulfat- und Alkylethersulfattensiden, berechnet auf die Gesamttensidmenge der Reinigungszusammensetzung,
c) gegebenenfalls eine dritte Reinigungszusammensetzung, die reinigende Tenside in einer Gesamtkonzentration im Bereich von 5 bis 50 Gew.-%, berechnet auf die Gesamtmenge der Zusammensetzung, enthält,
wobei die Zusammensetzungen in der folgenden Reihenfolge angewendet werden:
i) zuerst Auftragen der Zusammensetzung von a) auf die Keratinfasern, Abspülen derselben, zweitens Auftragen der Zusammensetzung von b) mit einer Zeitverzögerung von höchstens 12 Stunden nach dem Auftragen von a), Abspülen derselben, und gegebenenfalls drittens Auftragen der Zusammensetzung von c) und anschließendes Abspülen derselben,
ii) zuerst Auftragen der Zusammensetzung a) auf die Keratinfasern, als zweites Auftragen der Zusammensetzung b), Abspülen der Zusammensetzungen a) und b) und gegebenenfalls als drittes Auftragen der Zusammensetzung c), gefolgt von deren Abspülen,
iii) zunächst das Mischen der Zusammensetzungen a) und b) unmittelbar vor dem Auftragen auf die Keratinfasern, das Auftragen der Mischung auf die Keratinfasern und das Abspülen der Mischung und gegebenenfalls das zweite Auftragen der Zusammensetzung von c) und das anschließende Abspülen.

2. Verfahren nach Anspruch 1, **dadurch gekennzeichnet, dass** ein oder mehrere kationische(s) Polymer(e) der Zusammensetzung a) ausgewählt ist/sind aus Polyquaternium 6, Polyquaternium 7, Polyquaternium 16, Polyquaternium 37 und/oder deren Salz(en), Polymethacrylamidopropyltrimoniumchlorid und Polymethacrylamidopropyltrimoniumchlorid/Acrylamid-Copolymer, vorzugsweise ist es Polyquaternium 37.

3. Verfahren nach Anspruch 1 und/oder 2, **dadurch gekennzeichnet, dass** die Konzentration an kationischen Polymeren mit einer Ladungsdichte von mindestens 3 meq/g in der Zusammensetzung a) im Bereich von 0.01 bis 10 Gew.-%, vorzugsweise 0.05 bis 8 Gew.-%, besonders bevorzugt im Bereich von 0.1 bis 6 Gew.-%, weiter bevorzugt im Bereich von 0.2 bis 2 Gew.-%, berechnet auf die Gesamtmenge der Zusammensetzung a), liegt.

4. Verfahren nach einem der vorhergehenden Ansprüche, **dadurch gekennzeichnet, dass** der pH-Wert der Zusammensetzung a) und/oder b) und/oder c) im Bereich von 1 bis 8, vorzugsweise im Bereich von 3 bis 6.5, besonders bevorzugt im Bereich von 4 bis 6, liegt/liegen.

5. Verfahren nach einem der vorhergehenden Ansprüche, **dadurch gekennzeichnet, dass** die Zusammensetzungen a) und/oder b) und/oder c) ein oder mehrere Tenside enthalten, die vorzugsweise aus nichtionischen, kationischen, anionischen und/oder zwitterionischen/amphoteren Tensiden ausgewählt sind.

6. Verfahren nach einem der vorhergehenden Ansprüche, **dadurch gekennzeichnet, dass** die amphoteren/zwitterionischen Tenside aus Alkylamidobetainen und/oder Alkylamidoalkylbetainen und/oder Sultaintensiden ausgewählt sind.

7. Verfahren nach einem der vorhergehenden Ansprüche, **dadurch gekennzeichnet, dass** das kationische Tensid ausgewählt ist aus quaternären Ammoniumtensiden und/oder Alkylamidoalkylamin-Tensiden.

8. Verfahren nach einem der vorhergehenden Ansprüche, **dadurch gekennzeichnet, dass** die Gesamtkonzentration der Tenside der Zusammensetzungen b) und/oder c) im Bereich von 8 bis 35 Gew.-%, vorzugsweise im Bereich von 12 bis 25 Gew.-%, besonders bevorzugt im Bereich von 14 bis 20 Gew.-% liegt, berechnet auf die Gesamtmenge jeder der Zusammensetzungen b) und c), und die Gesamtkonzentration der Tenside in der Zusammensetzung a) unter 3 Gew.-%, vorzugsweise unter 2 Gew.-% liegt, berechnet auf die Gesamtmenge der Zusammensetzung a).

9. Verfahren nach einem der vorhergehenden Ansprüche, **dadurch gekennzeichnet, dass** die kationische Ladungsdichte des kationischen Polymers der Zusammensetzung a) mindestens 4.5 meq/g, vorzugsweise mindestens 5.5 meq/g beträgt.

10. Verfahren nach einem der vorhergehenden Ansprüche, **dadurch gekennzeichnet, dass** die Zusammensetzungen b) und/oder gegebenenfalls c) frei von kationischen Polymeren mit einer Ladungsdichte von mindestens 3 meq/g sind.

11. Verfahren nach einem der vorhergehenden Ansprüche, **dadurch gekennzeichnet, dass** die Zusammensetzungen a) und/oder b) und/oder gegebenenfalls c) eine oder mehrere hydrophobe Verbindung(en), vorzugsweise ausgewählt aus Fettalkoholen, Fettsäureestern, Silikonölen, aminierten Silikonen und Mineralöl, enthalten.

12. Verfahren nach einem der vorhergehenden Ansprüche, **dadurch gekennzeichnet, dass** die Zusammensetzung c) Alkylsulfat- und/oder Alkylethersulfat-Tenside in einer Konzentration von mehr als 50 Gew.-%, vorzugsweise mehr als 90 Gew.-%, bezogen auf die gesamte Tensidkonzentration der Zusammensetzung c), enthält.

## Revendications

1. Procédé de traitement des fibres kératiniques, de préférence des fibres kératiniques humaines, plus préférentiellement des cheveux humains, **caractérisé en ce qu'**il comprend les compositions suivantes :
a) une première composition non nettoyante comprenant un ou plusieurs polymère(s) cationique(s) et/ou leur(s) sel(s) avec une densité de charge d'au moins 3 meq/g, ayant une concentration totale en tensioactifs inférieure à 3% en poids, de préférence inférieure à 2% en poids, calculée par rapport au poids total de la composition a),
b) une deuxième composition nettoyante comprenant des agents de surface nettoyants à une concentration totale comprise entre 5% et 50% en poids, calculée par rapport au poids total de la composition nettoyante, et comprenant moins de 50% en poids, de préférence moins de 30% en poids, plus préférentiellement moins de 15% en poids d'agents de surface de type sulfate d'alkyle et sulfate d'éther d'alkyle, calculée par rapport à la quantité totale d'agents de surface de la composition nettoyante,
c) éventuellement, une troisième composition nettoyante comprenant des agents tensioactifs nettoyants à une concentration totale comprise entre 5% et 50% en poids, calculée par rapport à la quantité totale de la composition,
les compositions sont appliquées dans l'ordre suivant
i) premièrement, application de la composition a) sur les fibres kératiniques et rinçage, deuxièmement, application de la composition b) avec un délai maximum de 12 heures après l'application de la composition a), rinçage, et éventuellement troisièmement, application de la composition c) suivie d'un rinçage,
ii) appliquer d'abord sur les fibres kératiniques la composition a), appliquer ensuite la composition b), rincer les compositions a) et b), et éventuellement appliquer en troisième lieu la composition c) suivie d'un rinçage,
iii) mélanger d'abord les compositions a) et b) directement avant l'application sur les fibres kératiniques, appliquer le mélange sur les fibres kératiniques et rincer le mélange, et éventuellement appliquer ensuite la composition c) suivie d'un rinçage.

2. Méthode selon la revendication 1 **caractérisée par le fait qu'**un ou plusieurs polymères cationiques de la composition a) sont choisis parmi le Polyquaternium 6, le Polyquaternium 7, le Polyquaternium 16, le Polyquaternium 37, et/ou leur(s) sel(s), le chlorure de polyméthacrylamidopropyltrimonium, et le copolymère chlorure de polyméthacrylamidopropyltrimonium/acrylamide, de préférence il s'agit du Polyquaternium 37.

3. Méthode selon les revendications 1 et/ou 2 **caractérisée par le fait que** la concentration de polymères cationiques ayant une densité de charge d'au moins 3 meq/g dans la composition a) est comprise entre 0.01% et 10% en poids, de préférence entre 0.05% et 8% en poids, plus préférentiellement entre 0.1% et 6% en poids, encore plus préférentiellement entre 0.2% et 2% en poids, calculée par rapport à la composition totale a).

4. La méthode selon l'une des revendications précédentes est **caractérisée par le fait que** le pH de la composition a) et/ou b) et/ou c) est compris entre 1 et 8, de préférence entre 3 et 6.5, plus préférentiellement entre 4 et 6.

5. Méthode selon l'une quelconque des revendications précédentes, **caractérisée par le fait que** les compositions a) et/ou b) et/ou c) comprennent un ou plusieurs tensioactifs, de préférence choisis parmi les tensioactifs non ioniques, cationiques, anioniques et/ou zwitterioniques/amphotériques.

6. Méthode selon l'une quelconque des revendications précédentes, **caractérisée par le fait que** les agents de surface amphotères/zwitterioniques sont choisis parmi les alkylamido-bétaïnes et/ou les alkylamidoalkyl-bétaïnes et/ou les agents de surface sultaïnes.

7. Procédé selon l'une quelconque des revendications précédentes **caractérisé par le fait que** le tensioactif cationique est choisi parmi les tensioactifs à base d'ammonium quaternaire et/ou les tensioactifs à base d'alkylamidoalkylamines.

8. Méthode selon l'une quelconque des revendications précédentes, **caractérisée par le fait que** la concentration totale en tensioactifs des compositions b) et/ou c) est comprise entre 8% et 35% en poids, de préférence entre 12% et 25% en poids, plus préférentiellement entre 14% et 20% en poids, calculée par rapport au total de chaque composition b) et c), et que la concentration totale en tensioactifs de la composition a) est inférieure à 3% en poids, de préférence inférieure à 2% en poids, calculée par rapport au total de la composition a).

9. Méthode selon l'une quelconque des revendications précédentes, **caractérisée par le fait que** la densité de charge cationique du polymère cationique de la composition a) est d'au moins 4.5 meq/g, de préférence d'au moins 5.5 meq/g.

10. Procédé selon l'une quelconque des revendications précédentes **caractérisé par le fait que** les compositions b) et/ou éventuellement c) sont exemptes de polymères cationiques ayant une densité de charge d'au moins 3 meq/g.

11. Procédé selon l'une quelconque des revendications précédentes **caractérisé en ce que** les compositions a) et/ou b) et/ou éventuellement c) comprennent un ou plusieurs composé(s) hydrophobe(s), de préférence choisi(s) parmi les alcools gras, les esters d'acides gras, les huiles de silicone, les silicones aminées et les huiles minérales.

12. Méthode selon l'une quelconque des revendications précédentes, **caractérisée par le fait que** la composition c) comprend des tensioactifs de type sulfate d'alkyle et/ou éther sulfate d'alkyle à une concentration supérieure à 50% en poids, de préférence supérieure à 90% en poids, par rapport à la concentration totale en tensioactifs de la composition c).
